Europäisches Patentamt

European Patent Office

Office européen des brevets

(11) Numéro de publication: **0 554 322 B1**

# (12) FASCICULE DE BREVET EUROPEEN

(45) Date de publication de fascicule du brevet: **07.06.95**  (51) Int. Cl.[6]: **A61K 7/48**, A61K 35/78

(21) Numéro de dépôt: **91918857.3**

(22) Date de dépôt: **21.10.91**

(86) Numéro de dépôt internationale :
**PCT/FR91/00824**

(87) Numéro de publication internationale :
**WO 92/06673 (30.04.92 92/10)**

Le dossier contient des informations techniques
présentées postérieurement au dépôt de la
demande et ne figurant pas dans le présent
fascicule.

(54) **COMPOSITIONS DERMATOLOGIOUES ET/OU COSMETIOUES COMPRENANT DE LA GOMME AMMONIAOUEE ET UN EXTRAIT DE GRAINES DE PEGANUM HARMALIS.**

(30) Priorité: **22.10.90 FR 9013050**

(43) Date de publication de la demande:
**11.08.93 Bulletin 93/32**

(45) Mention de la délivrance du brevet:
**07.06.95 Bulletin 95/23**

(84) Etats contractants désignés:
**CH DE ES GB IT LI SE**

(56) Documents cités:
**FR-A- 2 502 008**

**S.T.N. Serveur de bases de données, Karlsruhe, DE; File Biosis, access number AN-
81:138348, M.E-S. El Rifaie: "Peganum-harmala its use in certain dermatoses", & Int. J.
Dermatoö. 19 (4), 1980**

**S.T.N. Serveur de bases de données, Karlsruhe, DE; File Biosis, access number AN-
85-300154, L.V- Belova: "Peganum-harmala in
the treatment of some dermatoses, & Vestn.
Dermatol. Venerol, 0 (7), 1984**

(73) Titulaire: **CHEKROUN, Mehir
4, allée Pierre-Gaspard
F-94400 Vitry (FR)**

(72) Inventeur: **CHEKROUN, Mehir
4, allée Pierre-Gaspard
F-94400 Vitry (FR)**

EP 0 554 322 B1

## Description

La présente Invention est relative à des compositions dermatologiques et/ou cosmétiques.

Les produits destinés à l'entretien et aux soins de la peau font actuellement l'objet de nombreuses recherches ; le but de ces préparations peut être le soin de différentes maladies de peau, l'aide à la cicatrisation, ou, plus simplement, la protection de la peau, son embellissement, ainsi que la prévention et l'atténuation des effets du vieillissement.

Il est donc toujours souhaitable de disposer de nombreux principes actifs pouvant être incorporés dans de tels produits.

L'Inventeur a déjà proposé l'utilisation, dans la Demande de Brevet Français 81 05487, de la gomme ammoniaque (gomme résine sécrétée par des ombellifères du genre *Dorema ammoniacum* Don., *et Dorema aucheri* Boiss.), pour l'obtention de compositions pharmaceutiques destinées au traitement de diverses maladies tumorales.

L'Inventeur a maintenant constaté que la gomme ammoniaque pouvait être associée à un extrait obtenu à partir des graines de la plante *Peganum harmalis*, de la famille des Rutacées, pour l'obtention de compositions cosmétiques et/ou dermatologiques.

Dans l'art antérieur, des extraits de *Peganum harmalis* ont été utilisés dans un but pharmaceutique, pour le traitement des maladies de peau [L.V. BELOVA, VESTN. DERMATOL. VENEROL. 0(7), 56-58 (1984)], et en particulier pour le traitement de certaines dermatoses inflammatoires [EL RIFAIE, INT.J. DERMATOL. 19(4), 221-222, (1980)].

Or l'Inventeur a constaté que des compositions résultant de l'association de gomme ammoniaque et d'extrait obtenu à partir des graines de la plante *Peganum harmalis* possèdent des propriétés cosmétiques et/ou dermatologiques. En particulier, ces compositions, appliquées localement sur la peau ou sur le cuir chevelu, possèdent de façon surprenante, la propriété d'améliorer l'état et l'aspect de la peau et des cheveux.

Les graines de *Peganum harmalis* sont vendues chez les marchands d'épices et de produits exotiques, sous l'appellation de "*Harmel*" ou de "*grain de mi*".

La présente Invention a pour objet des compositions pour l'usage cosmétique et/ou dermatologique, caractérisées en ce qu'elles comprennent, en tant que principe actif, une quantité appropriée de distillat, ou d'extrait alcoolique de gomme ammoniaque, associée à une quantité appropriée de distillat ou d'extrait alcoolique de graines de *Peganum harmalis*.

Selon un mode de réalisation préféré des compositions conformes à l'Invention, elles comprennent entre 1 et 10% en poids, de distillat ou d'extrait alcoolique de gomme ammoniaque et entre 1 à 10% en poids de distillat ou d'extrait alcoolique de graines de *Peganum harmalis*.

Selon une disposition préférée de ce mode de réalisation, les compositions conformes a l'Invention comprennent entre 2 et 6% en poids de distillat ou d'extrait alcoolique de gomme ammoniaque.

Selon une autre modalité préférée de ce mode ce réalisation, les compositions conformes à l'Invention comprennent entre 2 et 6% en poids de distillat ou d'extrait alcoolique de graines de *Peganum harmalis*.

Conformément à l'Invention, la gomme ammoniaque peut être utilisée dans un grand nombre de formulations destinées à l'usage cosmétique, telles que des crèmes, pommades, produits démaquillants, lotions, poudres, savons, shampooings, produits de maquillage ayant également un effet traitant, pâtes dentifrice, etc...

Pour l'obtention de compositions conformes à l'Invention, la gomme ammoniaque et l'extrait de graines de *Peganum harmalis* peuvent être associés à d'autres principes actifs, ainsi qu'à des excipients, conservateurs, agents de texture, parfums, couramment utilisés dans la formulation des produits cosmétiques.

Selon un mode de réalisation préféré des compositions conformes à l'Invention, elles comprennent en outre un corps gras.

Selon une disposition préférée de ce mode de réalisation, ledit corps gras est une huile d'origine végétale, telle que l'huile de sésame, de noix, de catharme, de tournesol.

Selon une modalité préférée de cette disposition, les compositions conformes à l'Invention comprennent entre 2 et 10%, en poids, d'huile de sésame.

La présente Invention sera mieux comprise à l'aide du complément de description qui va suivre, qui se réfère à des exemples de préparation des compositions cosmétiques conformes à l'Invention.

Il doit être bien entendu toutefois que ces exemples sont donnés uniquement à titre d'illustration de l'objet de l'Invention dont ils ne constituent en aucune manière une limitation.

**Exemple 1 : Préparation d'une crème dermique:**

```
Extrait éthanolique de gomme ammoniaque              3 g
Harmel* (extrait éthanolique)                        2 g
Huile de sésame                                      6 g
Parfum                                               1 g
```

**Base pour crème composée de :**

```
Cutina MD                    9 g
Eumulgade F                  3 g
Eutanol G                    8 g
Huile de maïs                8 g
Huile de vaseline            6 g
Lanosoluble AS               5 g
Phénonip                     0,2 g
Eau              q.s.p. 100 g
```

```
.................................................q.s.p    100 g
* (DUCROS - 26170 BUIS LES BARONNIES)
```

**Exemple 2 : Préparation d'un shampooing:**

```
Extrait éthanolique de gomme ammoniaque              6 g
Harmel (extrait éthanolique)                         6 g
Huile de sésame                                      2 g
Parfum                                               1 g
```

**Base pour shampooing composée de :**

```
Texapon 40                   300 g
Comperlan KD                  40 g
Huile de ricin hydrosoluble   30 g
Chlorure de sodium            15 g
Eau              q.s.p.  1000 g
```

```
.................................................q.s.p    100 g
```

**Exemple 3 : Préparation d'une pâte dentifrice:**

| | |
|---|---|
| Extrait éthanolique de gomme ammoniaque | 6 g |
| Harmel (extrait éthanolique) | 4 g |
| Argile rose ou blanche (ghassoul) | 50 g |
| Savon dentifrice neutre q.s.p. | 100 g |

EP 0 554 322 B1

**Exemple 4 : Préparation d'une lotion capillaire:**

```
Extrait éthanolique de gomme ammoniaque              5 g
Harmel (extrait éthanolique)                         2 g
Huile de sésame                                      5 g
Parfum                                               1 g
Base pour lotion capillaire composée de :
Panthénol                        0,125 g
Tween 80                         0,250 g
Vitamine F soluble               0,050 g
Soufre soluble                   0,125 g
Alcool à 90°                      163 ml
Eau q.s.p.                        250 ml
.................................................q.s.p    100 g
```

**Exemple 5 : Préparation d'une lotion pour la peau :**

Pour un litre de lotion, on fait distiller avec de l'eau 60 g de gomme ammoniaque, et on additionne au distillat 6 g d'extrait de harmel.

Ainsi que cela ressort de ce qui précède, l'Invention ne se limite nullement à ceux de ses modes de mise en oeuvre, de réalisation et d'application qui viennent d'être décrits de manière plus explicite ; elle en embrasse au contraire toutes les variantes qui peuvent venir à l'esprit du technicien en la matière, sans s'écarter du cadre, ni de la portée de la présente Invention.

**Revendications**

1. Compositions pour l'usage cosmétique et/ou dermatologique, caractérisées en ce qu'elles comprennent, en tant que principe actif, du distillat ou de l'extrait alcoolique de gomme ammoniaque, associé à du distillat ou de l'extrait alcoolique de graines de *Peganum harmalis*.

2. Compositions selon la revendication 1, caractérisées en ce qu'elles comprennent entre 1 et 10% en poids, de distillat ou d'extrait alcoolique de gomme ammoniaque, et entre 1 à 10% en poids de distillat ou d'extrait alcoolique de graines de *Peganum harmalis*.

3. Compositions selon l'une quelconque des revendications 1 ou 2, caractérisées en ce qu'elles comprennent entre 2 et 6% en poids, de distillat ou d'extrait alcoolique de gomme ammoniaque, et entre 2 et 6% en poids de distillat ou d'extrait alcoolique de graine de *Peganum harmalis*.

4. Compositions selon l'une quelconque des revendications 1 à 3, caractérisées en ce qu'elles comprennent en outre au moins un corps gras.

5. Compositions selon la revendication 4, caractérisées en ce que ledit corps gras est une huile d'origine végétale.

6. Compositions selon la revendication 5, caractérisées en ce qu'elles comprennent entre 2 et 10% en poids, d'huile de sésame.

4

**Claims**

1.  Compounds for cosmetic and/or dermatological usage, characterized by the fact that they include as an active principle, distillate or alcohol extract from ammonia gum, associated to distillate or alcohol extract from *Peganum harmalis* seeds.

2.  Compounds in claim number 1, characterized by the fact that they include from 1 to 10% of distillate or alcohol extract of ammonia gum in weigh and between 1 to 10% of distillate or alcohol extract of *Peganum harmalis* seeds in weigh.

3.  Compounds according to any one of the claims number 1 or 2, characterized by the fact that they include from 2 to 6% of distillate or alcohol extract of ammonia gum in weigh and between 2 to 6% of distillate or alcohol extract of *Peganum harmalis* seeds in weigh.

4.  Compounds according to any one of the claims 1 to 3, characterized by the fact that they also include a fat matter.

5.  Compounds according to claim 4, characterized by the fad that the fat matter is a plant-based-oil.

6.  Compounds according to claim 5, characterized by the fact that they contain between 2 and 10% of sesame oil in weigh.


**Patentansprüche**

1.  Zusammensetzungen zum Zwecke einer kosmetischen und/oder dermatologischen Verwendung, dadurch gekennzeichnet, daß diese Zusammensetzungen als hauptsächlichen Wirkstoff ein Destillat oder einen alkoholhaltigen Extrakt aus Ammoniakgummi verbunden mit einem Destillat oder einem alkoholhaltigen Extrakt aus Harmalkraut-Körnern aufweisen.

2.  Zusammensetzungen gemäß Anspruch 1), dadurch gekennzeichnet, daß diese zwischen 1 und 10% des Gewichts eines Destillats oder eines alkoholhaltigen Extrakts aus Ammoniakgummi und zwischen 1 und 10% des Gewichts eines Destillats oder eines alkoholhaltigen Extrakts aus Harmalkraut-Körnern ausmachen.

3.  Zusammensetzungen gemäß eines beliebigen Anspruchs nach 1) oder 2), dadurch gekennzeichnet, daß sie zwischen 2 und 6% des Gewichts eines Destillats oder eines alkoholhaltigen Extrakts aus Ammoniakgummi und zwischen 2 und 6% des Gewichts eines Destillats oder eines alkoholhaltigen Extrakts aus Harmalkraut-Körnern ausmachen.

4.  Zusammensetzungen gemäß eines beliebigen Anspruchs nach 1) bis 3), dadurch gekennzeichnet, daß sie mindestens einen Fettstoff aufweisen.

5.  Zusammensetzungen gemäß Anspruch 4), dadurch gekennzeichnet, daß besagter Fettstoff aus einem Öl pflanzlichen Ursprungs besteht.

6.  Zusammensetzungen gemäß Anspruch 5), dadurch gekennzeichnet, daß sie Sesamöl zwischen 2 und 10% des Gewichts aufweisen.